# EUROPEAN PATENT SPECIFICATION

(11) **EP 2 841 023 B1**
(45) Date of publication and mention of the grant of the patent: **13.12.2017**
(21) Application number: 12717713.7
(22) Date of filing: 27.04.2012
(51) Int. Cl.: A61F 2/30, A61F 2/44, A61F 2/46, A61B 17/02

(54) **INTERVERTEBRAL IMPLANT AND INSTRUMENT FOR USE IN PLACING IT**
ZWISCHENWIRBELIMPLANTAT UND INSTRUMENT ZUM EINSETZEN
IMPLANT VERTÉBRAL ET INSTRUMENT À UTILISER POUR LE PLACER

(43) Date of publication of application: 04.03.2015
(73) Proprietor: LFC Spólka Z.O.O., 65-364 Zielona Góra (PL)
(72) Inventor: CIUPIK, Lechoslaw Franciszek, PL-65-364 Zielona Góra (PL)
(74) Representative: PATERIS Patentanwälte PartmbB
(86) International application number: PCT/EP2012/057781
(87) International publication number: WO 2013/159823

(56) References cited:
- WO-A1-2004/000177
- WO-A2-96/40016
- US-A1- 2008 082 169
- US-A1- 2011 077 738
- US-B1- 6 491 695

## Description

The present invention relates to an intervertebral implant which can be used for a surgical correction of a sliding of vertebrae. Moreover, the present invention relates to an instrument for placing such an implant.

Literature data indicate that sliding of vertebrae, called spondylolisthesis, is found on average in 10% of patients treated because of back pain. This pathology may cause a significant disability, both among the youth and adult. Surgical treatment of spondylolisthesis is provided when neurological symptoms and/or pain occur, or increase, even though conservative treatment had been previously administered. From the state of the art it is known to correct a sliding of vertebrae by means of a surgical instrument used for displacing vertebrae relative to each other. Then, after the sliding of the vertebrae has been corrected by use of the instrument an implant is placed for stabilizing the vertebrae in the corrected position. Such surgical procedures and the relating instruments are, for example, described in US 5,601,556, US 5,69,977, US 6,491,695 B1, US 6,533,791 B1, US 2004/0073214 A1, US 2008/0319481 A1 and US 2010/0024487 A1.

In addition, surgical methods are known in which the implants are also used for correcting a sliding of vertebrae in a surgical treatment of spondylolisthesis. Such implants and instruments for placing the implants are, for example, disclosed in US 2007/0123989 A1, US 2009/0125062 Aand US 2011/0077738 A1.

In particular, US 2011/0077738 A1describes an implant which allows a surgical displacement of vertebrae for correcting a sliding of vertebrae in a treatment of spondylolisthesis. The intervertebral implant disclosed in this document comprises a first and a second implant body which extend along a longitudinal direction. The implant bodies are connected such as to be movable relative to each other along the longitudinal direction. The relative movement is imparted by a screw seated in one of the implant bodies and cooperating with a thread in the other one of the implant bodies. Hence, by a rotational movement of the screw, a linear movement of the implant bodies relative to each other can be effected.

WO 2004/000177 A1 describes a instrument for placement of a cage between vertebral bodies of a spinal column. The instrument includes an element with a hexagonal face at the end of a rod. The hexagonal face cooperates with a hexagonal face which is part of a bore through which the rod extends in order to prevent the rod from rotating within the bore.

With respect to the mentioned prior art it can be seen as an objective of the present invention to a provide an advantageous intervertebral implant which can be used in treating spondylolisthesis, as well as to provide an advantageous surgical instrument for placing the intervertebral implant during a surgical spondylolisthesis treatment.

The first objective is achieved by an intervertebral implant according to claim 1 and a surgical instrument according to claim 9, respectively.

According to a first aspect of the present invention, an intervertebral implant extending along a longitudinal direction is provided which comprises:
- A first implant body which extends along the longitudinal direction of the implant and includes a proximal end, called first proximal end in the following, and a distal end, called first distal end in the following, where the first proximal end and the first distal end form opposite ends of the first implant body in longitudinal direction.
- A second implant body which extends along the longitudinal direction of the implant and includes a proximal end, called second proximal end in the following, and a distal end, called second distal end in the following, where the second proximal end and the second distal end form opposite ends of the second implant body in longitudinal direction.
- A threaded member which is seated in the first implant body so as to be rotatable along a rotary axis extending in the longitudinal direction of the implant and which has a proximal end that is accessible from the outside of the implant. The proximal end of the threaded member comprises ant interface having a design which allows the transmission of a rotary motion about the rotary axis to the threaded member by use of a rotatable tool. Such an interface may, e.g., have the form of a male or female screw drive profile or any other profile that allows transferring a torque to the threaded member. The interface is called first interface in the following.
- A thread located in the second implant body which thread extends in longitudinal direction of the implant and is located such as to cooperate with the treaded member seated in the first implant body.
The first implant body and the second implant body are connected such as to be movable relative to each other along the longitudinal direction. Moreover, the thread in the second implant body is arranged to cooperate with the treaded member in the first implant body in order to impart a longitudinal movement to the second implant body relative to the first implant body upon a rotation of the threaded member about its rotary axis. The proximal end of the threaded member further comprises a securing means which allows for engagement of a mating securing element of a tool used for transmitting a rotary motion to the threaded member, so as to secure the proximal end from slipping off from the tool in longitudinal direction.

By providing a securing means in the proximal end of the threaded member which securing means allows for engagement of a mating securing element of a tool it becomes possible to secure the implant against slipping off from a tool used for placing the implant. At the same time, providing the securing means in the proximal end of the threaded member allows for keeping the instrument compact since the securing element of the instrument can be in very close proximity to the coupling interface of the tool. In particular, the interface and the securing element of the instrument may be arranged coaxially.

The state of the art documents US 2007/0123989 A1 and US 2011/0077738 A1, dealing with surgical implants which allow for a displacement of vertebrae, are silent if and how the implants are secured against slipping off from the tool used for imparting the rotary motion to the treated member. US 2009/0125062 Aproposes to use an electrical motor located in the implant for imparting the rotary motion so that securing of a tool is not necessary. Use of an electrical motor may, however, not be feasible in all cases. Moreover, use of an electrical motor may make the implant more expensive than an implant that is hand driven by use of a surgical instrument with a suitable tool.

The securing means of the inventive implant may be a threaded blind hole in the first interface which is aligned with the longitudinal direction of the implant. Alternatively, the securing means may be a threaded pin that is located at the first interface and aligned with the longitudinal direction of the implant. As a further alternative, the securing means may be a male or female part of a bayonet coupling.

Furthermore, the inventive implant may comprise an interface having a design which allows the transmission of a rotary motion to the whole implant by use of a mating interface of a surgical instrument. The ability to rotate the whole implant body is useful in in the surgical process of placing the intervertebral implant as will be described later. This interface is called second interface in the following. Such a second interface may also be present in implants which do not include the above securing means.

The second interface may, e.g., be implemented by at least one recess in a face of the first implant body which face extends from the first proximal end to the first distal end of the first implant body, where the recess extends in said face in longitudinal direction from said first proximal end towards said first distal end. Alternatively or additionally the second interface may include at least one recess in a face of the second implant body which face extends from the second proximal end to the second distal end of the second implant body, where the recess extends in said face in longitudinal direction from said second proximal end towards said second distal end. Due to the extension of the recess from the proximal end towards the distal end such an implementation of the second interface can also provide a guiding means for the implant bodies when they move relative to each other along the longitudinal direction.

In a first particular example of an implementation of the second interface, the first implant body includes a first side face that extends from the first proximal end to the first distal end, and a second side face that extends from the first proximal end to the first distal end and is located opposite to the first side face. A recess is present in each of the first side face and the second side face of the first implant body. Moreover, the second implant body includes a first side face that extends from the second proximal end to the second distal end, and a second side face that extends from the second proximal end to the second distal end and is located opposite to the first side face. A recess is present in each of the first side face and the second side face of the second implant body. Each of the recesses in the respective side faces begins at the respective first and second proximal end and extends in longitudinal direction towards the respective first and second distal end. In such an implementation of the second interface the forces acting on the implant to provide a torque for rotating the whole implant act on both implant bodies so that force differences between the first and second implant body can be avoided or at least reduced.

In an advantageous development of the first particular example, the recess in the first side face of the first implant body and the recess in the first side face of the second implant body each have the same depth and an open side where the open sides are open towards the respective other recess. In addition, the recess in the second side face of the first implant body and the recess in the second side face of the second implant body each have the same depth and an open side where the open sides are open towards the respective other recess. In this development, a projection of a mating coupling interface of an instrument for manipulating the implant can at the same time engage the recesses of both the first implant body and the second implant body. Moreover, if all recesses have a flat bottom face, and the bottom faces of the recesses in the first implant body are aligned with the bottom faces of the recesses in the second implant body the projection in the mating coupling interface may have a flat face as coupling face which flat face can engage the flat bottom face of the recesses. Hence, the projection of the mating coupling interface can have a simple structure.

In a further development of the first particular example, the combined width of the recess in the first side face of the first implant body and the recess in the first side face of the second implant body differs from the combined width of the recess in the second side face of the first implant body and the recess in the second side face of the second implant body. This further development is advantageous if the implant is to be fixed to the instrument used during the surgical procedure in a defined orientation. Then, the different widths of the recesses can cooperate with projections of the instrument which have different widths to prevent the implant to be fixed in the wrong orientation.

In a second particular example of an implementation of the second interface, the first implant body includes at least one notch in the first proximal end and/or at least one notch in the second proximal end. Only having notches in the proximal end of an implant body allows the projections that are present in an instrument for engaging the notches of the implant to be located closer to the tool for rotating the threaded member of the implant. Hence, compared to the first particular example of an implementation of the second interface the second particular example allows to reduce the width of the mating second coupling interface of an instrument used during the surgical procedure.

To allow for fixing the implant to the vertebrae between which it is located the first implant body and the second implant body can be equipped with drive-anchoring elements, e.g., in form of sharp edges extending perpendicular to the first and second side faces of the implant or in form of sharp edges extending helically along the circumference of the implant so that the drive anchoring elements run in a thread-like fashion along the implant.

According to a second aspect of the present invention, a surgical instrument for use in placing an inventive intervertebral implant between two vertebrae and/or correcting a sliding of vertebrae is provided. The instrument comprises a proximal end, a distal end that is spaced from the proximal end in a longitudinal direction, a handle located at the proximal end, a manipulator section located at the distal end, and a hollow rod extending along the longitudinal direction from the handle to the manipulator section. The manipulator section comprises a rotatable tool with a coupling interface, e.g. in the form of a male or female screw drive profile or any other profile that allows for transferring a torque, which coupling interface allows for coupling to a mating first interface at the proximal end of a threaded element of an intervertebral implant and for the transfer of a rotary motion about a rotary axis extending in longitudinal direction of the instrument to the threated element of the intervertebral implant. This coupling interface is called first coupling interface in the following. The manipulator section further comprises a securing element which is designed to engage a mating securing means at the proximal end of the threaded member of the intervertebral implant so as to secure the tool from slipping off from the proximal end of the threaded member along a longitudinal direction.

The inventive instrument can be used together with the inventive implant in the surgical method that will be described later. In particular, the implant can be fixed to the manipulator section using the securing element of the instrument and the securing means of the implant, and the longitudinal movement of the first implant body relative to the second implant body can be imparted by a coupling of the first coupling interface of the instrument (which coupling interface is located at the rotatable tool) and the first interface of the intervertebral implant (which is located in treated member of the implant). Hence, when the implant is fixed to the instrument the movement of the first implant body relative to the second implant body can be accomplished by rotating the tool of the manipulator section of the instrument.

In a first example of the securing element this element is implemented as a threaded pin that is located at the first coupling interface, e.g. in form of a pin projecting over the first coupling interface or in form of a pin located in a recess in the first coupling interface, and aligned with the longitudinal direction of the instrument. In a second example of the securing element this element is implemented as a sleeve with a threaded blind hole which is aligned with the longitudinal direction of the instrument, where the sleeve is located at the first coupling interface. The pin or the sleeve is advantageously rotatable about a rotary axis that extends in longitudinal direction of the instrument where the pin or the sleeve is arranged such that it can be rotated without at the same time rotating the rotatable tool. In a third example of the securing element this element is implemented as a male or female part of a bayonet coupling.

If the tool of the surgical instrument is rotatable about its rotary axis by means of a hollow first drive shaft running through the hollow rod from the handle to the tool, and the pin or the sleeve is rotatable about its rotary axis by means of a second drive shaft running through the hollow first drive shaft from the handle to the pin or the sleeve, respectively, a compact arrangement with minimal lateral dimensions can be realized.

The manipulator section of the surgical instrument may comprise a coupling interface that allows coupling to a mating second interface of the implant. This coupling interface is called second coupling interface in the following. Then, the implant and the instrument can be coupled to each other by means of the second interfaces and the second coupling interface so as to allow the implant to be rotated as a whole by means of the coupling thus provided, while the coupling achieved by the first interface of the implant and the first coupling interface of the manipulator section allows to rotate a single part of the implant, namely the threaded member, so as to impart the relative movement between the first implant body and the second implant body. The second coupling interface may be present in the manipulator section of the inventive instrument even if the above securing element is not present.

The second coupling interface may be formed by at least one projection which projects in longitudinal direction and is spaced from the tool in a direction perpendicular to the longitudinal direction. In particular, the coupling interface may be formed by a first projection and a second projection of the manipulator section which both project in longitudinal direction, which are spaced from the tool in a direction perpendicular to the longitudinal direction, and which are located at opposite sides of the tool. If a first and a second projection are present, the width of first projection may differ from the width of the second projection, which allows for preventing the implant from being fixed to the instrument in a wrong orientation by giving recesses in the implant which are adapted to being engaged by the projections different widths which correspond to the different widths of the projections.

With the inventive implant and the inventive instrument, a surgical method of correcting a sliding of vertebrae can be performed. In this method, at least a first inventive intervertebral implant is placed between two vertebrae to be corrected by means of an inventive instrument. The sliding of the vertebrae is corrected by moving the second implant body relative to the first implant body along the longitudinal direction of the implant. According to the method, placing the intervertebral implant between the two vertebrae is done by mounting an intervertebral implant to the manipulator section of the instrument such that the first coupling interface of the tool of the instrument engages a mating first interface at the proximal end of the threaded member of the implant, and then introducing the intervertebral implant into a space between the two vertebrae using the instrument with the implant mounted thereto. Moving the second implant body relative to the first implant body along the longitudinal direction of the implant is done by rotating the threaded body of the implant through rotating the tool of the instrument.

If the mounting the implant is done by use of a securing means of the implant and a securing element of the manipulator section of the instrument a single manipulator section of the instrument can be used at the same time for securing the implant to the instrument and for moving the implant bodies relative to each other.

Placing the intervertebral implant between the two vertebrae may be done by introducing the intervertebral implant with its side faces showing towards the two vertebrae and then rotating the whole respective intervertebral implant by a defined angle, e.g. an angle in a range from 80 to 100 degree, in particular by 90 degree, about its longitudinal direction. This rotation can be imparted to the implant by the instrument through a coupling of a second interface of the implant to a second coupling interface of the manipulator section of the instrument. Throughout this rotation drive-anchoring elements in form of sharp edges can cut into the bone of the vertebrae. In order to make the cutting process more gentle, rotating the respective intervertebral implant by the defined angle may be done by repeated forth and back rotations where the rotation angle increases with each successive forth rotation until the defined angle is reached.

Although the implant can be introduced into the intervertebral space in the open or closed state there may be reasons to introduce it in the open state. In this case, moving the second implant body relative to the first implant body along the longitudinal direction of the implant is done by moving the second implant body forth relative to the first implant body along the longitudinal direction of the implant by a defined amount before the intervertebral implant is placed between the two vertebrae and then, after the implant is placed between the two vertebrae, moving the second implant body back relative to the first implant body along the longitudinal direction of the implant by the same defined amount by which it was moved forth before it was placed between the two vertebrae. By this measure, the implant is in the closed state after the sliding of the vertebrae has been corrected, thus providing a high stability. Please note that moving the second implant body forth relative to the first implant body along the longitudinal direction of the implant by a defined amount before the intervertebral implant is placed between the two vertebrae can either be done before or after mounting the implant to the instrument.

Although correcting a displacement of vertebrae may be done with a single implant in some cases it may be advantageous to use two implants that are placed at opposite sides of the spinal cord. In this case, a first inventive intervertebral implant is placed between two vertebrae to be adjusted by means of an inventive instrument, and a second inventive intervertebral implant is placed between the same two vertebrae to be adjusted by means of an inventive instrument. The sliding of the vertebrae is adjusted by moving the second implant body relative to the first implant body along the longitudinal direction of the implant in the first intervertebral implant as well as in the second intervertebral implant.

Further features, properties and advantages will become clear from the following description of embodiments of the invention in conjunction with the accompanying drawings.
- Figure 1: shows a first embodiment of an inventive intervertebral implant in a partially cut away perspective view.
- Figure 2: shows a second embodiment of the inventive intervertebral implant in a perspective view.
- Figure 3: shows an embodiment of a screw which can be used as threaded member in an intervertebral implant.
- Figure 4: shows a cut through the screw of Figure 5.
- Figure 5: shows a third embodiment of the inventive intervertebral implant in a perspective view.
- Figure 6: shows a fourth embodiment of the inventive intervertebral implant in a perspective view.
- Figure 7: shows a second embodiment of a screw which can be used as a threaded member in an intervertebral implant in a perspective view.
- Figure 8: shows a cut through the screw of Figure 7.
- Figure 9: shows a third embodiment of the screw which can be used us a threaded member of the inventive intervertebral implant in a perspective view.
- Figure 10: shows a cut through the screw of Figure 9.
- Figure 11: shows the inventive instrument in a plan view.
- Figure 12: shows the manipulator section of the instrument shown in Figure 11 in a perspective view.
- Figure 13: shows an alternative manipulator section of the inventive instrument in a perspective view.
- Figure 14: shows a first step of the method of correcting a sliding of vertebrae.
- Figure 15: shows a second step of the method of correcting a sliding of vertebrae.
- Figure 16: shows a third step of the method of correcting a sliding of vertebrae.
- Figure 17: shows a fourth step of the method of correcting a sliding of vertebrae.

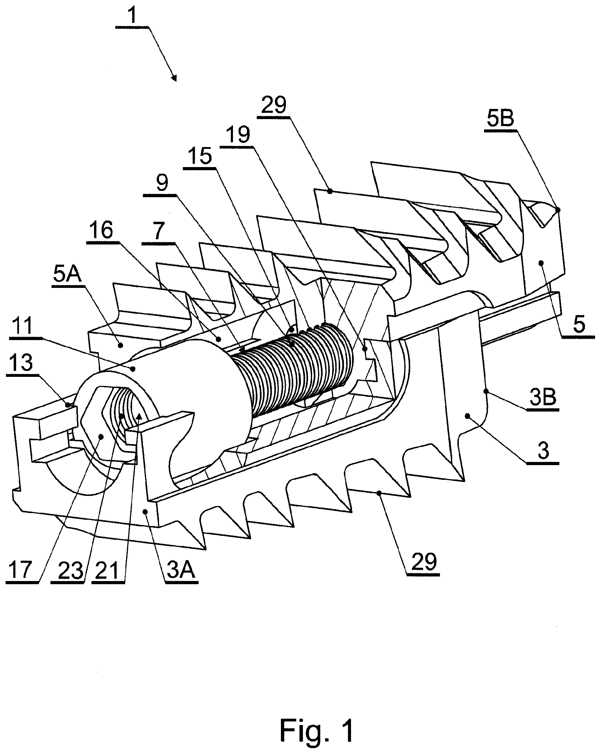

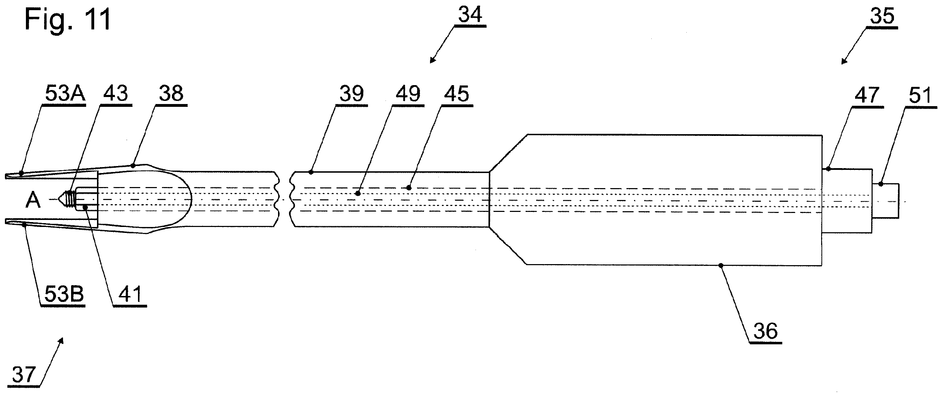

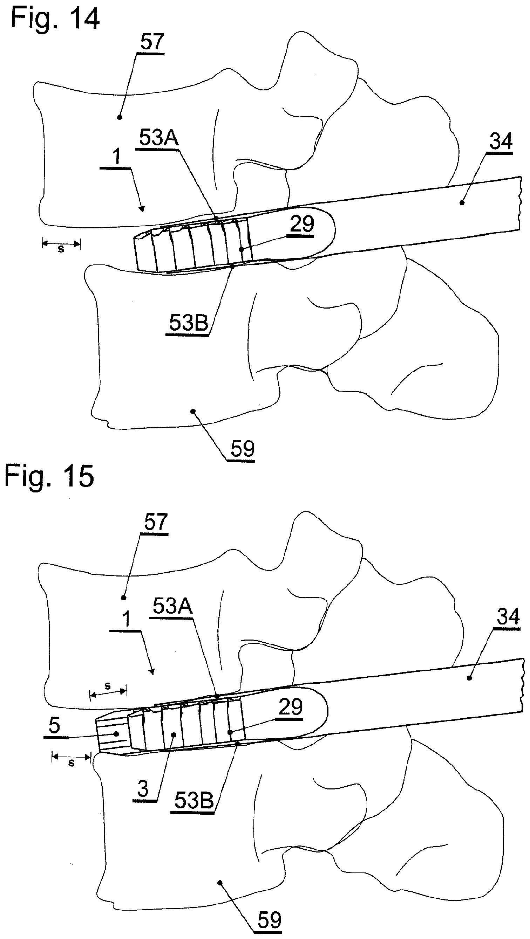

In the following sections particular embodiments of the invention will be described with respect to the accompanying Figures. While Figures 1 to 10 relate to the inventive implant Figures 11 to 13 relate to the inventive instrument for placing the implant and Figures 14 to 17 relate to the method of correcting a sliding of vertebrae. Please note that an intervertebral implant together with a corresponding surgical instrument that is adapted to the respective intervertebral implant are intended to be used together and, hence, form a closely related surgical system.

The following description of specific embodiments will focus on the intervertebral implant first and then step on to the description of embodiments of the surgical instrument which can be used for placing the implant. After the implant and the instrument have been described the use of a system comprising an implant and a relating surgical instrument for correcting a sliding of vertebrae will be described.

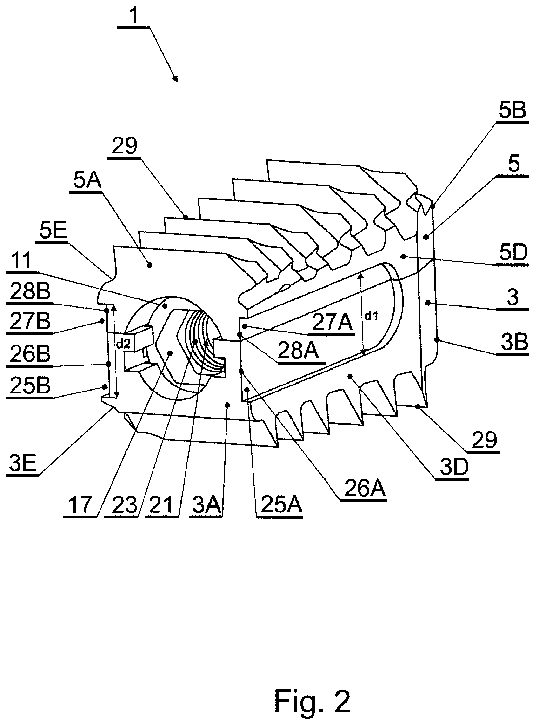

A first embodiment of the intervertebral implant is shown in Figures 1 and 2. While Figure 1 shows a perspective view in which the implant is partially cut away and the implant is in an open state Figure 2 shows a perspective view onto the implant of Figure 1 in a closed state.

As shown in Figure 1, the inventive intervertebral implant 1 is composed of three main parts, namely a first implant body 3, a second implant body 5, and a threaded member 7 in form of a screw comprising a threaded shaft 9 and a screw head 11. The screw is located in the first implant body 3, i.e. the first implant body includes a seat 13 accommodating the screw head 11.

The second implant body 5 is equipped with a bore 15 with an internal thread that cooperates with the external thread of the screw shaft 9. Moreover, the second implant body 5 comprises a proximal end 5A with an enlarged bore 16 that is large enough for accommodating the screw head 11 therein and the length of which is larger than the extension of the screw head 11 in the longitudinal direction of the screw 7. The length enlarged bore 16 defined the distance by which the second implant body 5 can travel with respect to the longitudinal direction of the screw 7. The longitudinal direction of the screw 7 also defines the longitudinal direction of the implant 1.

The proximal end of the implant 1 is the end where the screw head 11 is located; the distal end of the implant is the opposite end of the implant. As a consequence, likewise the proximal ends 3A, 5A of the first and second implant bodies 3, 5 are those ends in which the screw head 11 is located, their distal ends 3B,5B are those ends which are located opposite to the ends with the screw head 11.

The proximal end 3A of the first implant body 3 as well as the proximal end 5A of the second implant body 5 are open towards the seat of the screw head so that a tool which can engage an interface in the screw head 11 can be put through the distal end of the implant 1 to reach the interface 17 in the screw head 11.

The second implant body 5 is connected to the first implant body 3 in such a way that it can move in longitudinal direction with respect to the first implant body 3. This longitudinal movement can be imparted to the second implant body 5 by rotating the screw 7 whereby the external thread in the screw shaft 9 cooperates with the internal thread in the bore 15 of the second implant body 5 so that upon rotation of the screw 7 the second implant body 5 moves longitudinally with respect to the first implant body 3. A guiding mechanism, which is implemented in the form of a tongue and a groove joint 19 in the present embodiment, prevents the second implant body 5 from moving relative to the first implant body 3 in a direction perpendicular to the longitudinal direction.

In the present embodiment, the interface 17 in the screw head is a hexagonal opening. However, it could be any other suitable screw drive profile like square opening, slotted profiles including cruciform types, star-like profiles, etc. The only restriction to the shape of the interface is that the shape must allow transferring a rotary motion to the screw by use of a tool. In other words, the interface must comprise faces that allow transferring of momentum to the screw. Moreover, although the screw head of the present embodiment is equipped with the female part of a screw drive profile it could as well be equipped with the male part of a profile. In this case, the instrument used for transferring to the rotary motion to the screw would be equipped with the female part of the profile.

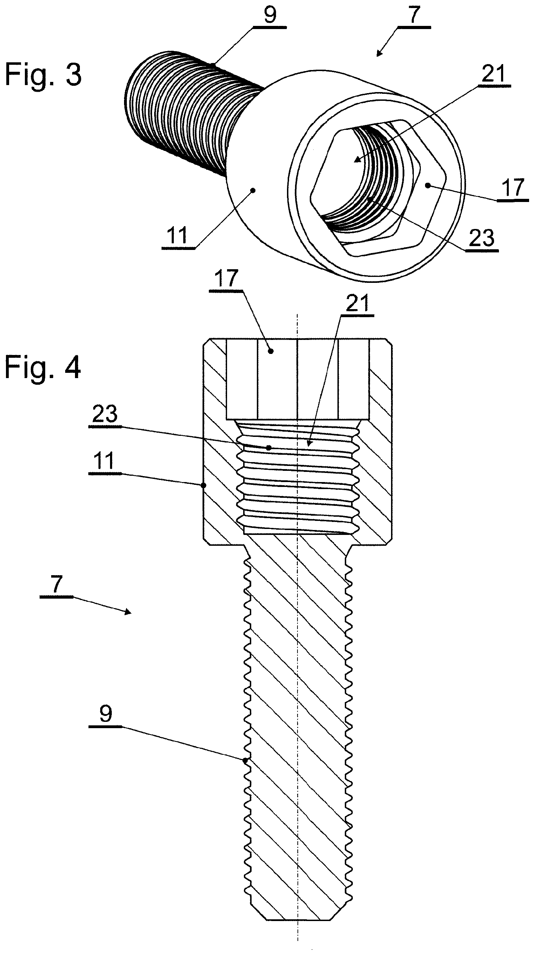

As can be best seen in Figures 3 and 4, a blind hole 21 is provided at the bottom of the hexagonal opening 17 in the screw head 11 which blind hole 21 comprises an internal thread 23. The blind hole 21 with the internal thread 23 serves as a securing means for securing the tool by which the rotary motion is imparted to the screw 7 to the screw head 11 when the implant is mounted to the instrument, so as to prevent the tool form slipping off from the interface 17. The tool that will be used together with the present embodiment of the intervertebral implant comprises a threaded pin that forms a mating securing element of the tool that can be screwed into the blind hole 21 as will be explained later with respect to the inventive surgical instrument. Please note that other securing means than internal threads can be used in the inventive implant. Of course, the mating securing element of the instrument needs to be adapted to the respective securing means of the implant. For example, a bayonet type connection is conceivable where the screw head comprises the male or female part of the coupling and the tool comprises the respective other part of the coupling.

In the embodiment shown in Figures 1 and 2 the implant has a generally oblong cross section. This means the first implant body 3 and the second implant body 5 each comprise first and second side faces 3D and 3E, and 5D and 5E, respectively, which are parallel to each other, extend from the respective proximal ends 3A, 5A to the respective distal ends 3B, 5B of the implant bodies and are located on opposite sides of the screw 7.

The side faces 3D, 3E of the first implant body 3 each comprise a recess 25A, 25B that begins at the proximal end 3A and extends in a longitudinal direction towards the distal end 3B. The recess in each side face 3D, 3E has a flat bottom face 26A, 26B and is open towards the second implant body 5.

Likewise, the second implant body 5 is equipped with recesses 27A, 27B which begin at the proximal end 5A of the second implant body and extend along the longitudinal direction towards the distal end 5b of the second implant body. These recesses also have flat bottom faces 28A, 28B which are aligned with the flat bottom faces 26A, 26B of the recesses in the side faces 3D, 3E of the first implant body 3. Moreover, the recesses of an implant body are open towards the recesses of the respective other implant body. The length of the recesses 27A, 27B in the side faces 5D, 5E of the second implant body correspond to the length of the recesses 25A, 25B in the side faces 3D, 3E of the first implant body. Hence, when the implant is closed as it is shown in Figure 2 the recesses in the side faces of the first implant body 3 and the recesses in the side faces second implant body 5 together form two recesses in the implant 1. The recesses, in particular their bottom faces 26A, 26B, 28A, 28B, form a second interface which can cooperate with a second coupling interface of the surgical instrument so as to allow rotating the whole implant 1 about the longitudinal axis. The meaning of this rotation will be explained later with respect to the surgical method.

As can be seen from Figure 2, the combined width of the recesses 25A, 27A in the first side faces 3D, 5D of the implant 1 differs from the combined width of the recesses 25B, 27B in the second side faces 3E, 5E of the implant 1. By this measure it can be assured that the implant 1 will be fixed to the surgical instrument in the correct orientation.

For anchoring the implant 1 in the vertebrae between which it is placed the implant comprises drive anchoring elements 29. In the present embodiment these drive anchoring elements 29 are formed as sharp edges extending perpendicular to the longitudinal direction of the implant 1 and perpendicular to the side faces 3D, 3E, 5D, 5E of the implant bodies 3, 5. Each sharp edge has a triangular cross section that has a first limit which is more or less perpendicular to the longitudinal direction and a second limit that includes a different angle than 90 degree with the longitudinal direction. While the perpendicular limits show towards the distal end 3B in the first implant body 3 they show towards the proximal end 5A in the second implant body 5.

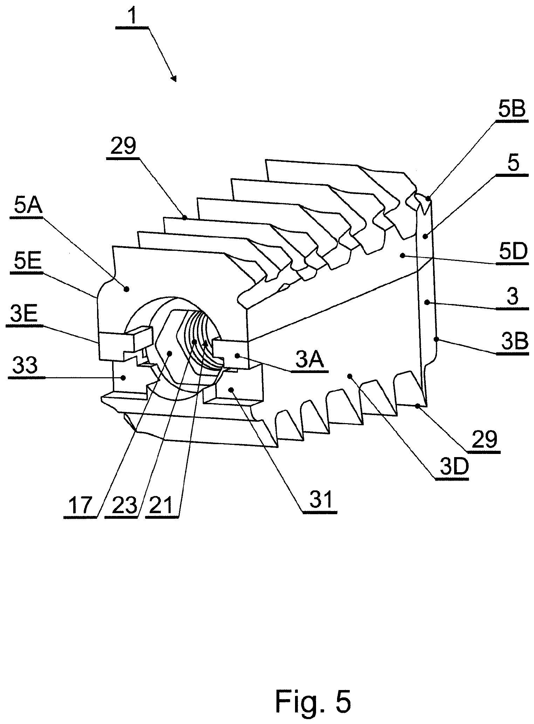

Figure 5 shows a modification of the embodiment that has been described with respect to Figures 1 to 4. Elements that do not differ from the first embodiment are denominated with the same reference numerals as in Figures 1 to 4 and will not be described again to avoid repetitions. Hence, the description of the second embodiment concentrates on the differences with respect to the first embodiment.

The difference of the second embodiment, shown in Figure 5, to the first embodiment, shown in Figures 1 to 4, lies in that no recesses 25A, 25B, 27A, 27B are present in the side faces 3D, 3E, 5D, 5E. Instead, recesses or notches 31, 33 are present in the proximal ends 3A of the first implant body 3. Like the recesses in the first embodiment, the notches 31, 33 in the proximal end 3A of the first implant body 3 of the present embodiment serve as a second interface that allows to transfer a rotary motion to the whole implant 1 by a suitable coupling interface of the surgical instrument. By providing the notches 31, 33 only in the first implant body fixing the implant 1 to the surgical instrument in a wrong orientation can be avoided.

While the configuration of the second interface of the first embodiment is advantageous in that a higher momentum can be transferred to the implant and in that it can also serve as a guide means during the relative movement between the implant bodies the configuration of the interface in the second embodiment is advantageous in that the second coupling interface of the surgical instrument can have more compact lateral dimensions.

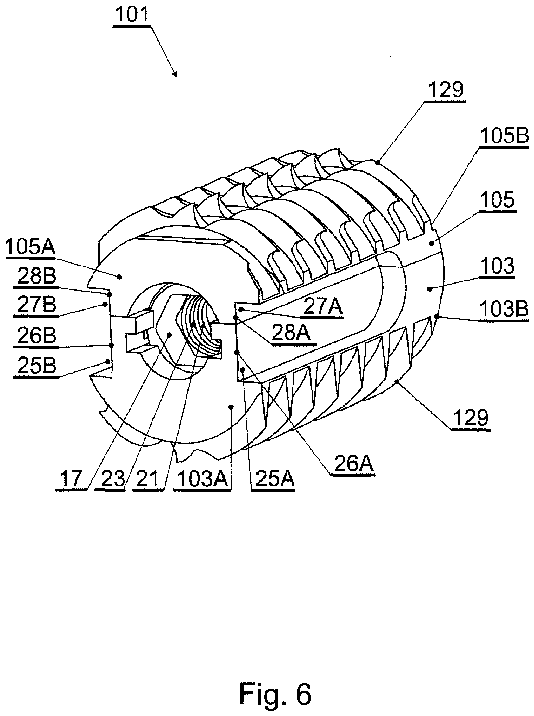

A third embodiment of the intervertebral implant is shown in Figure 6. Again, those elements that do not differ from the first embodiment are denominated by the same reference numerals as in Figures 1 to 4 and will not be described again. Thus, the description of the third embodiment will focus on the differences with respect to the first embodiment.

The intervertebral implant of the third embodiment mainly differs from the intervertebral implant of the first embodiment in that its cross section perpendicular to the longitudinal direction is more or less circular instead of being oblong. Hence, the implant 101 of the third embodiment has a more or less cylindrical circumferential face that is equipped with anchoring elements in form of sharp edges 129. Moreover, the sharp edges 129 extend helically around the circumferential face of the implant 101 so that a thread-like configuration of the anchoring elements is achieved. The almost circular cross section can be best seen when looking at the proximal ends 103A, 105A of the first and second implant bodies 103, 105 of the implant 101 according to the third embodiment.

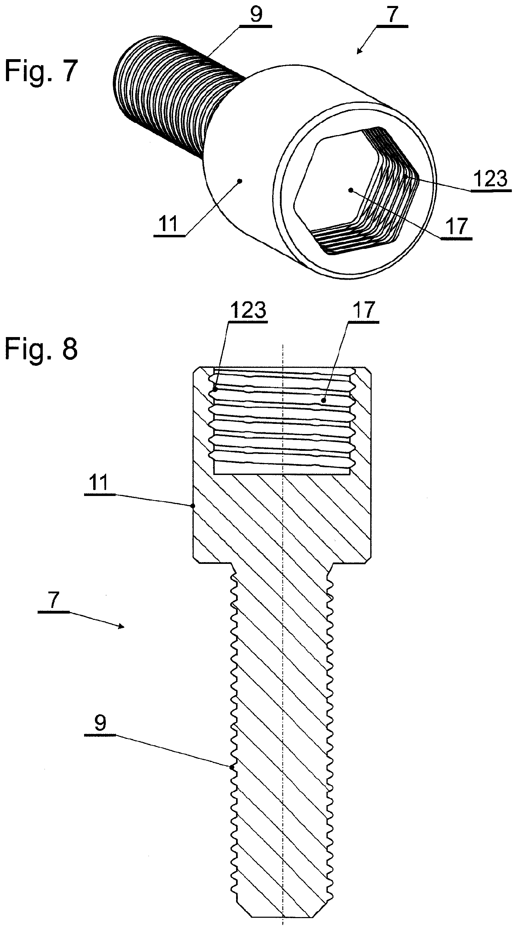

The first to third embodiments have been described to comprise screws with a hexagonal opening as interface and a threaded blind hole as securing means. With respect to Figures 7 to 10 different configurations of the interface and/or the securing means will be described.

As screw with a screw head comprising a hexagonal interface like in the first embodiment is shown in Figures 7 and 8. However, in difference to the screw of the first embodiment the securing means is not implemented as blind hole in the bottom face of the hexagonal opening 17 but as a thread 123 cut into the side faces of the hexagonal opening 17.

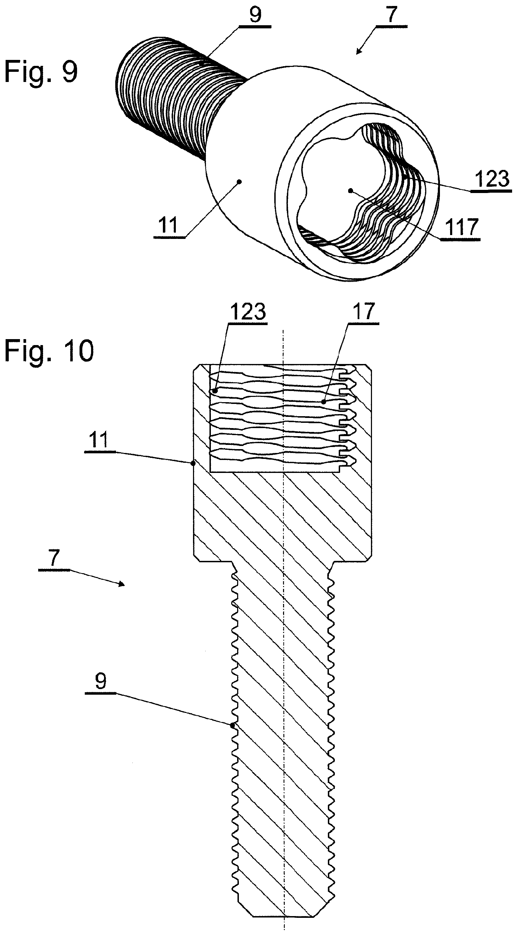

The screw shown in Figures 9 and 10 differs from the screw shown in Figures 7 and 8 in that its interface 117 has a different shape than a hexagonal opening. Like in the screw shown in Figures 7 and 8 a internal thread 123 is cut into the side faces of the interface 117.

Please note that the shown interfaces and securing means are only illustrative examples of a large number of possible interfaces and securing means.

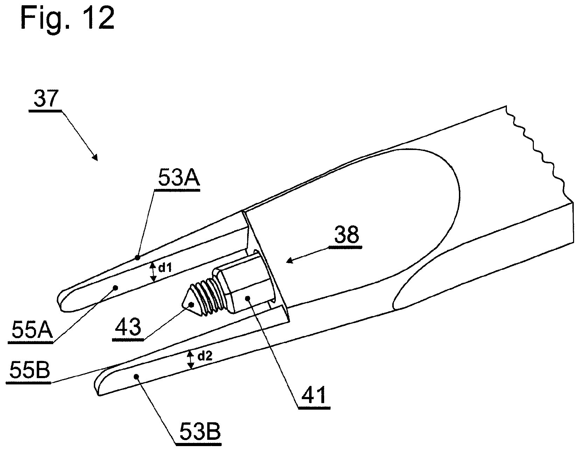

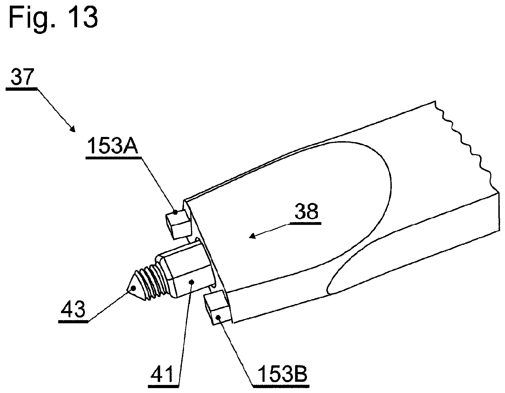

An inventive surgical instrument 34 will now be described with respect to Figures 11 to 13. While Figures 11 and 12 show a surgical instrument that is adapted to be used with the first and third embodiments of the intervertebral implant the surgical instrument 34 shown in Figure 13 is adapted to be used with the second embodiment of the intervertebral implant.

The surgical instrument 34 according to the invention comprises a proximal end 35 with a handle 36 and a distal end 37 that is spaced from the proximal end in a longitudinal direction and comprises a manipulator section 38. The manipulator section 38 is connected to the handle 36 by means of a hollow rod 39 that extends along the longitudinal direction from the manipulator section 38 to the handle 36.

The manipulator section 38 is equipped with a first coupling interface 41 that is provided by a hexagonal cylinder, i.e. a cylinder with a hexagonal cross-section, in the present embodiment. The cylinder is hollow, and a threaded pin 43 is located in the opening of the hollow hexagonal cylinder. Moreover, the threaded pin 43 can project over the hexagonal cylinder. The threaded pin 43 is adapted to be screwed into the threaded blind hole of the head 11 of the screw 7 that forms the threaded member in the first and third embodiments and, hence, forms the securing element by which the tool, i.e. the hexagonal coupling interface, can be secured to the proximal end of the threaded member, i.e. to the screw head 11.

A hollow drive shaft 45 extends from a knob 47 located at the handle 36 through the handle 36 and the hollow rod 39 to the hexagonal cylinder 41 and is connected to the cylinder so that the cylinder rotates together with the hollow drive shaft 45 when the shaft is rotated by means of the knob 47.

A second drive shaft 49 is located inside the hollow drive shaft 45 and extends from a second knob 51 at the handle 36 through the hollow drive shaft 45 to the threaded pin 43. The second drive shaft 49 is fixed to the threaded pin 43 so that the pin can be rotated by rotating the second knob 51 at the handle 36. In particular, the described configuration allows the pin 43 to be rotated without at the same time rotating the hexagonal cylinder 41.

The manipulator section 37 further comprises a second interface which is provided by two projections 53A, 53B the widths d1, d2 (see Figure 12) of which correspond to the widths of the combined recesses in the first side faces of the implant and the second side faces of the implant, respectively. The projections 53A, 53B each have flat contact faces 55A, 55B for contacting the flat bottom faces of the recesses in the intervertebral implant. These contact faces 55A, 55B show towards the longitudinal axis A of the instrument 34. They form an interface by means of which the whole implant can be rotated through rotating the inventive surgical instrument 34 about its longitudinal axis.

An alternative configuration of the manipulator section 38 is shown in Figure 13. Elements that do not differ from the configuration of the manipulator section shown in Figures 11 and 12 are denominated with the same reference numerals as in Figures 11 and 12 and will not be explained again.

The manipulator section 38 according to the second configuration differs from the manipulator section according to the first configuration shown in Figures 11 and 12 in the shape of the projections 153A, 153B projecting from the manipulator section 38. As can be seen by comparing Figures 12 and 13, the projections of the second configuration are much shorter than the projections of the first configuration. Like in the first configuration they are located at opposite sides with respect to the first interface 41. However, the lateral outer surfaces of the projections are located closer to the longitudinal axis A than in the first configuration which allows reducing the lateral dimensions of the manipulator section. The projections 153A, 153B of the second configuration are adapted to engage the notches 31, 33 of the second embodiment of the inventive intervertebral implant. Hence, the instrument 134 of the second embodiment, which is shown in Figure 13, is adapted for being used together with the intervertebral implant of the second embodiment of the implant.

Although the instrument is only shown with a hexagonal cylinder as first interface other shapes of the first interface are possible. For example, the exterior surface of the cylinder can be adapted to the shape of the interface 117 in the screw head shown in Figures 9 to 12. In addition, other polygonal cross sections of the cylinder are also conceivable, for example three- or four-sided cross sections. Moreover, the coupling interface could also be in the form of a screw driver for a slit profile in a screw head. In addition, instead of a threaded pin a bayonet-like securing element, a rotatable sleeve with an internal thread, etc. could be used. In the latter case, a pin with an external thread would be located for example in the center of the hexagonal opening of the screw head shown in Figure 3.

The use of the intervertebral implant and the surgical instrument for correcting a sliding of vertebrae will be described with respect to Figures 14 to 17. These Figures show different steps of a surgical procedure for treating spondylolisthesis. In this treatment a sliding of neighboring vertebrae is corrected and the vertebrae are stabilized by use of an intervertebral implant. In the depicted surgical method, the treatment is performed from posterior although it can, in general, also be performed from anterior. Moreover, in the embodiment of the method that is shown in Figures 14 to 17 an intervertebral implant 1 according to the first embodiment of the intervertebral implant and a surgical instrument 34 according to the first embodiment of the instrument are used. However, the other embodiments of the implant and the instrument could be used as well.

Figure 14 shows two vertebrae 57, 59 where a sliding of the upper vertebra 57 with respect to the lower vertebra 59 about the distance S has occurred. Hence, the upper vertebra 57 is displaced with respect to the lower vertebra 59 by the distance S which displacement will be corrected by the surgical method. In a first step, which is shown in Figure 14, an inventive intervertebral implant 1 mounted to the manipulator section 38 of an inventive surgical instrument 34 is introduced into the space between the two vertebrae 57, 59. As already mentioned, although introducing the intervertebral implant is done from posterior in the shown embodiment of the method, it can, in general, also be done from anterior.

When introducing the intervertebral implant, the implant 1 is placed beside the spinal cord. In the embodiment of the method shown in the Figures 14 to 17 only a single implant is placed at one side of the spinal cord. However, if necessary, a second intervertebral implant can be placed beside the spinal cord on the opposite side of the cord.

Introducing the intervertebral implant 1 into the space between the vertebrae 57, 59 is done in an orientation in which the side faces 3D, 5D, 3E, 5E of the implant 1 show towards the vertebrae 57, 59.

After the intervertebral implant has been placed between the vertebrae 57, 59 the implant is opened, i.e. the second implant body 5 is moved along the longitudinal direction relative to the first implant body 3 by rotating the screw 7. Rotating the screw 7 is done by means of the knob 47, by which the hexagonal coupling interface 41 that is coupled to the first interface 17 of the implant 1, i.e. the interface in the screw head 11, is rotated. Thereby, the rotation of the coupling interface 41 is transferred to the screw 7 seated in the first implant body 3. The screw then acts together with the thread in the second implant body 5 so as to move the second implant body 5 relative to the first implant body 3 in longitudinal direction. The implant 1 in the opened state is shown in Figure 15.

To prevent the implant 1 from slipping off the coupling interface 41 it is secured to the manipulator section 38 of the instrument by means of the pin 43 which engages the threaded blind hole 21 in the screw head 11. The pin 43 is brought into engagement with the threaded blind hole 21 in the screw head by rotating the second knob 51 at the handle 36 of the surgical instrument 34. Since the pin 43 can be rotated by means of the second knob 51 without, at the same time, rotating the first coupling interface 41 securing the intervertebral implant 1 to the manipulator section 38 of the instrument 34 can be done independently from rotating the screw 7 in the implant. In particular, the screw 7 can be held in a fixed rotational orientation by means of the knob 47. On the other hand, if the hollow drive shaft 45 for rotating the coupling interface 41 shows a higher resistance against rotation about the longitudinal axis of the instrument than the drive shaft 49 for rotating the threaded pin 43 it can be achieved that screwing the pin into the threaded blind hole of the screw head of the implant becomes possible without the necessity to keep the screw in a fixed rotational position by hand using the knob 47.

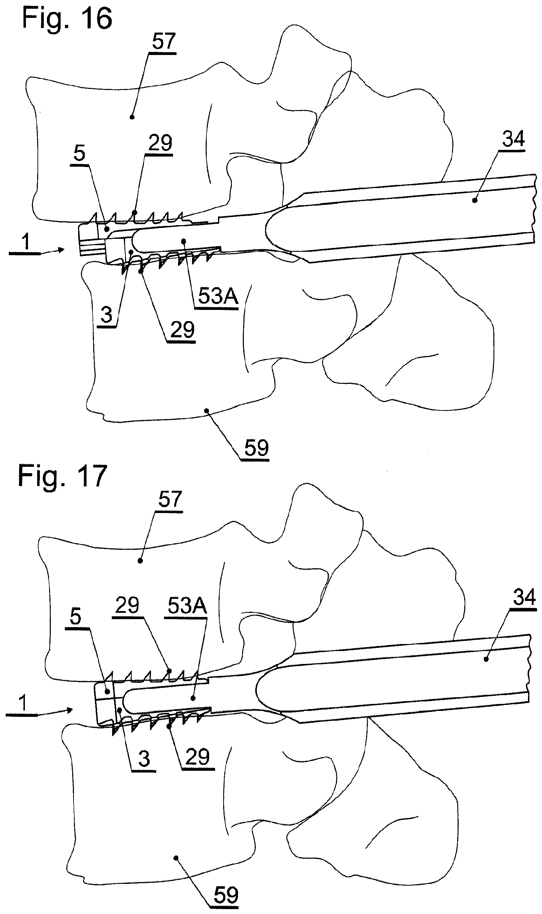

After the implant 1 has been opened by a distance S corresponding to the sliding distance of the vertebrae the implant is rotated by 90 degree to a position that is shown in Figure 16. Rotating the implant 1 is done by rotating the instrument 34. The rotation of the instrument 34 is transferred to the whole implant 1 by means of the second coupling interface of the instrument and the second interface of the implant, i.e. by the projections 53A, 53B the flat faces of which engage the flat bottom faces 26A, 26B, 28A, 28B of the recesses in the side faces of the implant 1. While the implant 1 is rotated about 90 degree the sharp edges of the drive anchoring elements 29 cut into the bone of the vertebrae 57, 59, thus anchoring the first and second implant bodies 3, 5 in the vertebrae 57, 59.

Advantageously, the cutting process, i.e. rotating the implant 1, it not done in a single step but by successive rotational forth and back movements where the rotation starts with an angle smaller than the final angle of 90 degree and where the angle of rotation increases which each forth rotational movement. A typical sequence could be rotating the implant forth from 0 degree to 15 degree and return to 0 degree in a backward rotational movement, then rotating the implant in a second forth movement to 30 degree and returning to 0 degree in a second back movement, then rotating the implant to 45 degree in a third forth movement and returning to 0 degree in a third back movement, then rotating the implant to 60 degree in a fourth forth movement and returning to 0 degree in a forth back movement, then rotating the implant to 75 degree in a fifth forth movement and returning the implant to 0 degree by a fifth back movement, and then rotating the implant to 90 degree in a sixth forth movement which is the last movement.

Please note that the rotation about 90 degree and the sequence of forth and back movements that have been described with respect to the present embodiment are not mandatory. The rotation can have a final angle smaller or larger than 90 degree, for example 85 degree or 95 degree. Typically the final rotation angle will be in a range of 80 to 100 degree. Moreover, while the angle of rotation increases by 15 degree in each forth rotation of the present embodiment the steps could be larger or smaller, for example 10 or 20 degree. Typically, the steps are in the range of 10 to 30 degree. Furthermore, it is not mandatory to return to 0 degree after each forward rotation. It is also possible to rotate the implant back by a defined fixed angle in each backwards rotation.

In case two implants are used in the surgical method it is either possible to rotate the first implant to the final angle, i.e. 90 degree in the present case, and then to rotate the second implant to the final angle. However, it is also possible to perform the first forth and back movement for the first implant, then performing the first forth and back movement of the second implant, then returning to the first implant for the second forth and back movement, and so on until both implants have been rotated to the final angle.

After the first and second implant bodies 3, 5 have been anchored in the vertebrae 57, 59, i.e. when the final angle of rotation is reached, the implant will be closed. In other words, the second implant body 5 is moved in longitudinal direction with respect to the first implant body 3 until the original configuration, i.e. the configuration before opening the implant, has been reached. Since the implant bodies 3, 5 are anchored in the vertebrae 57, 59 the vertebrae follow the relative movement between the first and second implant bodies 3, 5 whereby the sliding of the vertebrae is corrected. The final state of the implant and the vertebrae 57, 59 is shown in Figure 17.

If two implants are used in the surgical procedure the implant are either closed simultaneously if two surgeons performing the surgery or stepwise if a single surgeon is performing the surgery. If two intervertebral implants are closed stepwise the first implant is closed by a step that is small enough not to produce a dangerous twist between the two vertebrae 57, 59. Then, the second implant is closed either by the same amount or twice the amount before the surgeon returns to the first implant.

After the sliding of the vertebrae 57, 59 has been corrected the instrument is dismounted from the implant by use of the knob 51 for unscrewing the pin 43 of the instrument from the threaded blind hole 21 of the screw 7 in the implant 1. The implant then stays between the vertebrae.

Although the present invention has been described for illustrative reasons by means of specific embodiments the invention shall not be restricted to these embodiments since deviations from the shown embodiments are possible. For example, in the method, the implant 1 can be opened before fixing it to the instrument. If, for example a screw as shown in Figure 7 is used the implant could be opened by use of a hexagonal tool and, after it has been opened, fixed to the instrument by use of a threaded pin the dimensions of which are chosen such as to allow to screw the pin into the thread in the screw head. After the pin has been screwed into the opening of the screw head as far as it will go, a further rotation of the pin will impart a rotation to the screw 7. The orientation of the threads of the screw head and the pin are chosen such that the rotation imparted to the screw after the pin has been screwed in as far as it will go imparts a closing movement to the implant. Releasing the implant from the instrument after closing it can then be done by changing the orientation of rotation of the pin so that the pin is screwed out of the screw head again. This design allows providing the instrument with a rotatable threaded pin only. The pin then constitutes the securing means and, at the same time, the first coupling interface. Hence, an instrument with a single drive shaft is sufficient, which simplifies the mechanics of the instrument. In addition, it is not mandatory to open the implant before introducing it into the intervertebral space. It could also be introduced in the closed state and then opened when it is anchored in the vertebrae. Moreover, also deviations from the implants shown in the first to third embodiments are conceivable. For example, instead of forming a single recess the recess in the first side face of the first implant body and the recess in the first side face of the second implant body could stay separate so that two separate recesses would be formed in each side face of the implant. Moreover, the bottom face of a recess does not need to be flat but could have a different shape. By shaping the bottom faces of the recesses in one side face differently from the bottom faces in the other side face the implant can be prevented from being fixed to the instrument in a wrong orientation even if the widths and locations of the recesses in both side faces are identical. Of course, in this case the flat faces of the projections are replaced by a shape that corresponds to the shape of the bottom faces of the recesses. In addition, with respect to the embodiment shown in Figure 5 notches like the notches in the first implant body could also be present in the second implant body. By giving the notches different characteristics, like for example different sizes or different shapes, the implant can be prevented from being fixed to the instrument in a wrong orientation. Of course, the instrument shown in Figure 13 would be amended, in this case, to include four projections instead of two. Since various deviations from the described embodiments are possible the invention shall only be limited by the scope of the appended claims.

### Reference Numerals

- 1: intervertebral implant
- 3: first implant body
- 3A: proximal end
- 3B: distal end
- 3D: side face
- 3E: side face
- 5: second implant body
- 5A: proximal end
- 5B: distal end
- 5D: side face
- 5E: side face
- 7: screw
- 9: threaded shaft
- 11: screw head
- 13: seat
- 15: bore
- 16: enlarged bore
- 17: interface
- 19: guiding mechanism
- 21: blind hole
- 23: internal thread
- 25 A,B: recess
- 26 A,B: flat bottom face
- 27 A,B: recess
- 28 A,B: flat bottom face
- 29: anchoring elements
- 31: notch
- 33: notch
- 34: surgical instrument
- 35: proximal end
- 36: handle
- 37: distal end
- 38: manipulator section
- 39: rod
- 41: coupling interface
- 43: threaded pin
- 45: hollow drive shaft
- 47: knob
- 49: drive shaft
- 51: knob
- 53 A,B: projection
- 55 A,B: flat face
- 57: vertebra
- 59: vertebra
- 101: intervertebral implant
- 103: first implant body
- 103A: proximal end
- 103B: distal end
- 105: second implant body
- 105A: proximal end
- 105B: distal end
- 117: interface
- 123: thread
- 129: anchoring elements
- 153 A,B: projection

## Claims

1. An intervertebral implant (1,101) extending along a longitudinal direction, comprising;
- a first implant body (3,103) which extends along a longitudinal direction of the implant (1,101) and includes a first proximal end (3A,103A) and a first distal end (3B,103B) forming opposite ends of the first implant body (3,103) in longitudinal direction, and a second implant body (5,105) which extends along the longitudinal direction of the implant (1,101) and includes a second proximal end (5A,105A) and a second distal end (5B,105B) forming opposite ends of the second implant body (5,105) in longitudinal direction, where the first implant body (3,103) and the second implant body (5,105) are connected such as to be movable relative to each other along the longitudinal direction;
- a threaded member (7) which is seated in the first implant body (3,103) so as to be rotatable along a rotary axis extending in the longitudinal direction of the implant (1, 101) and which has a proximal end (11) that is accessible from the outside of the implant (1, 101), where the proximal end (11) of the threaded member (7) comprises a first interface (17,117) having a design which allows the transmission of a rotary motion about the rotary axis to the threaded member (7) by use of a rotatable tool; and
- a thread (15) located in the second implant body (5,105), which thread (15) extends in longitudinal direction of the implant (1, 101) and is arranged such as to cooperate with the treaded member (7) seated in the first implant body (3,103) in a way that the threaded member (7) imparts a longitudinal movement to the second implant body (5,105) relative to the first implant body (3,103) upon rotation of the threaded member (7) about its rotary axis;
**characterized in that**
the proximal end (11) of the threaded member (7) further comprises a securing means (23,123) which allows for engagement of a mating securing element of a tool used for transmitting a rotary motion to the threaded member (7), so as to secure the proximal end (11) from slipping off from the tool in longitudinal direction.

2. The intervertebral implant (1,101) as claimed in claim 1, **characterized in that** the first interface (17,117) is in the form of a male or female screw drive profile.

3. The intervertebral implant (1,101) as claimed in claim 1 or claim 2, **characterized in that** the securing means is a threaded blind hole (21) in the first interface (17) which is aligned with the longitudinal direction of the implant (1,101), or a threaded pin that is located at the first interface (17) and aligned with the longitudinal direction of the implant (1, 101), or the securing means is a male or female part of a bayonet coupling.

4. An intervertebral implant (1,101) as claimed in any of the claims 1 to 3, further comprising;
a second interface (25A,25B,27A,27B) having a design which allows the transmission of a rotary motion to the whole implant (1,101) by use of a mating interface of a tool.

5. The intervertebral implant (1) as claimed in claim 4, **characterized in that** the second interface is realized **in that**
- the first implant body (3,103) includes at least one recess (25A,25B) in a face (3D,3E) that extends from the first proximal end (3A,103A) to the first distal end (3B,103B), where the recess (25A,25B) extends in said face (3D,3E) in longitudinal direction from said first proximal end (3A,103A) towards said first distal end (3B,103B); and/or
- the second implant body (5,105) includes at least one recess (27A,27B) in a face (5D,5E) that extends from the second proximal end (5A,105A) to the second distal end (5B,105B), where the recess (27A,27B) extends in said face (5D,5E) in longitudinal direction from said second proximal end (5A, 105A) towards said second distal end (5B,105B).

6. The intervertebral implant (1) as claimed in claim 5, **characterized in that**
- the first implant body (3) includes a first side face (3D) that extends from the first proximal end (3A) to the first distal end (3B), and a second side face (3E) that extends from the first proximal end (3A) to the first distal end (3B) and is located opposite to the first side face (3D), where a recess (25A,25B) is present in each of the first side face (3D) and the second side face (3E) of the first implant body (3);
- the second implant body (5) includes a first side face (5D) that extends from the second proximal end (5A) to the second distal end (5B), and a second side face (5E) that extends from the second proximal end (5A) to the second distal end (5B) and is located opposite to the first side face (5D), where a recess (27A,27B) is present in each of the first side face (5D) and the second side face (5E) of the second implant body (5); and
- each of the recesses (25A,25B,27A,27B) in the respective side faces (3D,3E,5D,5E) begins at the respective first and second proximal end (3A, 5A) and extends in longitudinal direction towards the respective first and second distal end (3B, 5B).

7. The intervertebral implant (1) as claimed in claim 6, **characterized in that**
- the recess (25A) in the first side face (3D) of the first implant body (3) and the recess (27A) in the first side face (5D) of the second implant body (5) each have the same depth and an open side where the open sides are open towards the respective other recess,
- the recess (25B) in the second side face (3E) of the first implant body (3) and the recess (27B) in the second side face (3E) of the second implant body (5) each have the same depth and an open side where the open sides are open towards the respective other recess,
- that the combined width of the recess (25A) in the first side face (3D) of the first implant body (3) and the recess (27A) in the first side face (5D) of the second implant body (5) differs from the combined width of the recess (25B) in the second side face (3E) of the first implant body (3) and the recess (27B) in the second side face (5E) of the second implant body (5).

8. The intervertebral implant (1) as claimed in claim 4, **characterized in that** the second interface is realized **in that** the first implant body (3) includes at least one notch (31,33) in the first proximal end (3A) and/or at least one notch in the second proximal end (5A).

9. A surgical instrument (34) for use in placing an intervertebral implant according to any of the claims 1 to 8 between two vertebrae (57,59) and/or correcting a sliding of vertebrae (57,59), where the instrument (34) comprises a proximal end (35), a distal end (37) that is spaced from the proximal end (35) in a longitudinal direction, a handle (36) located at the proximal end (35), a manipulator section (38) located at the distal end (37), and a hollow rod (39) extending along the longitudinal direction from the handle (36) to the manipulator section (38), and where
- the manipulator section (38) comprises a rotatable tool with a first coupling interface (41) that allows for coupling to a mating first interface at the proximal end (11) of a threaded element (7) of an intervertebral implant (1,101) and for the transfer of a rotary motion about a rotary axis extending in longitudinal direction of the instrument (34) to the threaded element (7) of the intervertebral implant (1,101), and a securing element (43) which is designed to engage a mating securing means at the proximal end (11) of the threaded member (7) of the intervertebral implant (1,101) so as to secure the tool from moving away from the proximal end (11) of the threaded member (7) along a longitudinal direction.

10. The surgical instrument (34) as claimed in claim 9, in which the first coupling interface (41) is in the form of a male or female screw drive profile.

11. The surgical instrument (34) as claimed in claim 9 or claim 10, in which the securing element is a threaded pin (43) that is located at the first coupling interface (41) and aligned with the longitudinal direction of the instrument (34), or a sleeve with a threaded blind hole which is aligned with the longitudinal direction of the instrument (34), where the sleeve is located at the first coupling interface (41), or the securing element is a male or female part of a bayonet coupling.

12. A surgical instrument (34) as claimed in any of the claims 9 to 11, for use in placing an intervertebral implant (1,101) according to any of the claims 1 to 8 between two vertebrae (57,59) and/or correcting a sliding of vertebrae (57,59), further comprising a second coupling interface (53A,53B,153A,153B) that allows coupling to a mating second interface of the implant (1,101).

13. The surgical instrument (34) as claimed in claim 12, in which the second coupling interface is formed by at least one projection (53A,53B,153A,153B) of the manipulator section (38) which projects in longitudinal direction and is spaced from the tool (41) in a direction perpendicular to the longitudinal direction.

14. The surgical instrument (34) as claimed in claim 13 in which the coupling interface is formed by a first projection (53A, 153A) and a second projection (53B, 153B) of the manipulator section (38) which both project in longitudinal direction, which are spaced from the tool (41) in a direction perpendicular to the longitudinal direction, and which are located at opposite sides of the tool (41) and in which the width of first projection differs (53A,153A) from the width of the second projection (53B, 153B).

## Patentansprüche

1. Ein Zwischenwirbelimplantat (1, 101), das sich entlang einer Längsrichtung erstreckt, umfassend:
- einen ersten Implantatkörper (3, 103), der sich entlang einer Längsrichtung des Implantats (1, 101) erstreckt und ein erstes proximales Ende (3A, 103A) und ein erstes distales Ende (3B, 103B) einschließt, die entgegengesetzte Enden des ersten Implantatkörpers (3, 103) in Längsrichtung bilden, und einen zweiten Implantatkörper (5, 105), der sich entlang der Längsrichtung des Implantats (1, 101) erstreckt und ein zweites proximales Ende (5A, 105A) und ein zweites distales Ende (5B, 105B) einschließt, die entgegengesetzte Enden des zweiten Implantatkörpers (5, 105) in Längsrichtung bilden, wobei der erste Implantatkörper (3, 103) und der zweite Implantatkörper (5, 105) so verbunden sind, dass sie relativ zueinander entlang der Längsrichtung beweglich sind;
- ein Gewindeelement (7), das in dem ersten Implantatkörper (3, 103) so angeordnet ist, dass es entlang einer Drehachse, die sich in der Längsrichtung des Implantats (1, 101) erstreckt, drehbar ist, und das ein proximales Ende (11) aufweist, das von außerhalb des Implantats (1, 101) zugängig ist, wobei das proximale Ende (11) des Gewindeelements (7) einen ersten Verbindungsabschnitt (17, 117) umfasst, der ein Design aufweist, welches die Übertragung einer Drehbewegung um die Drehachse des Gewindeelements (7) durch Verwendung eines drehbaren Werkzeugs erlaubt; und
- einen Gewindegang (15), der in dem zweiten Implantatkörper (5, 105) liegt, wobei der Gewindegang (15) sich in Längsrichtung des Implantats (1, 101) erstreckt und so angeordnet ist, dass er mit dem im ersten Implantatkörper (3, 103) angeordneten Gewindeelement (7) in einer Weise zusammenwirkt, dass das Gewindeelement (7) durch Drehung des Gewindeelements (7) um seine Drehachse eine Längsbewegung des zweiten Implantatkörpers (5, 105) relativ zum ersten Implantatkörper (3, 103) herbeiführt;
**dadurch gekennzeichnet, dass**
das proximale Ende (11) des Gewindeelements (7) weiterhin ein Sicherungsmittel (23, 123) umfasst, welches den Eingriff eines passenden Sicherungselements eines Werkzeugs ermöglicht, das zum Übertragen einer Drehbewegung auf das Gewindeelement (7) verwendet wird, um das proximale Ende (11) vor dem Abrutschen von dem Werkzeug in Längsrichtung zu schützen.

2. Das Zwischenwirbelimplantat (1, 101) wie in Anspruch 1 beansprucht, **dadurch gekennzeichnet, dass** der erste Verbindungsabschnitt (17, 117) die Form eines männlichen oder weiblichen Profils zum Antreiben einer Schraube aufweist.

3. Das Zwischenwirbelimplantat (1, 101) wie in Anspruch 1 oder Anspruch 2 beansprucht, **dadurch gekennzeichnet, dass** das Sicherungsmittel ein Gewindesackloch (21) in dem ersten Verbindungsabschnitt (17) ist, welches in Längsrichtung des Implantats (1, 101) ausgerichtet ist, oder ein Gewindestift, der am ersten Verbindungsabschnitt (17) liegt und in Längsrichtung des Implantats (1, 101) ausgerichtet ist, oder das Sicherungsmittel ist ein männlicher oder weiblicher Teil eines Bajonettverschlusses.

4. Ein Zwischenwirbelimplantat (1, 101) wie in einem der Ansprüche 1 bis 3 beansprucht, weiterhin umfassend:
einen zweiten Verbindungsabschnitt (25A, 25B, 27A, 27B), der ein Design aufweist, das eine Übertragung einer Drehbewegung auf das gesamte Implantat (1, 101) durch Verwendung eines passenden Verbindungsabschnitts eines Werkzeugs erlaubt.

5. Das Zwischenwirbelimplantat (1) wie in Anspruch 4 beansprucht, **dadurch gekennzeichnet, dass** der zweite Verbindungsabschnitt dadurch realisiert ist, dass
- der erste Implantatkörper (3, 103) mindestens eine Vertiefung (25A, 25B) in einer Fläche (3D, 3E) einschließt, die sich von dem ersten proximalen Ende (3A, 103A) zu dem ersten distalen Ende (3B, 103B) erstreckt, wobei die Vertiefung (25A, 25B) sich in der Fläche (3D, 3E) in Längsrichtung von dem ersten proximalen Ende (3A, 103A) in Richtung auf das erste distale Ende (3B, 103B) erstreckt; und/oder
- der zweite Implantatkörper (5, 105) mindestens eine Vertiefung (27A, 27B) in einer Fläche (5D, 5E) einschließt, die sich von dem zweiten proximalen Ende (5A, 105A) zu dem zweiten distalen Ende (5B, 105B) erstreckt, wobei die Vertiefung (27A, 27B) sich in der Fläche (5D, 5E) in Längsrichtung von dem zweiten proximalen Ende (5A, 105A) in Richtung auf das zweite distale Ende (5B, 105B) erstreckt.

6. Das Zwischenwirbelimplantat (1) wie in Anspruch 5 beansprucht, **dadurch gekennzeichnet, dass**
- der erste Implantatkörper (3) eine erste Seitenfläche (3D), die sich von dem ersten proximalen Ende (3A) zu dem ersten distalen Ende (3B) erstreckt, und eine zweite Seitenfläche (3E), die sich von dem ersten proximalen Ende (3A) zu dem ersten distalen Ende (3B) erstreckt und gegenüber der ersten Seitenfläche (3D) liegt, einschließt, wobei eine Vertiefung (25A, 25B) sowohl in der ersten Seitenfläche (3D) als auch in der zweiten Seitenfläche (3E) des ersten Implantatkörpers (3) vorliegt;
- der zweite Implantatkörper (5) eine erste Seitenfläche (5D), die sich von dem zweiten proximalen Ende (5A) zu dem zweiten distalen Ende (5B) erstreckt, und eine zweite Seitenfläche (5E), die sich von dem zweiten proximalen Ende (5A) zu dem zweiten distalen Ende (5B) erstreckt und gegenüber der ersten Seitenfläche (5D) liegt, einschließt, wobei eine Vertiefung (27A, 27B) sowohl in der ersten Seitenfläche (5D) als auch in der zweiten Seitenfläche (5E) des zweiten Implantatkörpers (5) vorliegt; und
- jede der Vertiefungen (25A, 25B, 27A, 27B) in den entsprechenden Seitenflächen (3D, 3E, 5D, 5E) an dem entsprechenden ersten und zweiten proximalen Ende (3A, 5A) beginnt und sich in Längsrichtung in Richtung auf das jeweilige erste und zweite distale Ende (3B, 5B) erstreckt.

7. Das Zwischenwirbelimplantat (1) wie in Anspruch 6 beansprucht, **dadurch gekennzeichnet, dass**
- die Vertiefung (25A) in der ersten Seitenfläche (3D) des ersten Implantatkörpers (3) und die Vertiefung (27A) in der ersten Seitenfläche (5D) des zweiten Implantatkörpers (5) jeweils dieselbe Tiefe und eine offene Seite aufweisen, wobei die offenen Seiten offen in Richtung auf die jeweils andere Vertiefung sind,
- die Vertiefung (25B) in der zweiten Seitenfläche (3E) des ersten Implantatkörpers (3) und die Vertiefung (27B) in der zweiten Seitenfläche (3E) des zweiten Implantatkörpers (5) beide dieselbe Tiefe und eine offene Seite aufweisen, wobei die offenen Seiten offen in Richtung auf die jeweils andere Vertiefung sind,
- die kombinierte Breite der Vertiefung (25A) in der ersten Seitenfläche (3D) des ersten Implantatkörpers (3) und der Vertiefung (27A) in der ersten Seitenfläche (5D) des zweiten Implantatkörpers (5) sich von der kombinierten Breite der Vertiefung (25B) in der zweiten Seitenfläche (3E) des ersten Implantatkörpers (3) und der Vertiefung (27B) in der zweiten Seitenfläche (5E) des zweiten Implantatkörpers (5) unterscheidet.

8. Das Zwischenwirbelimplantat (1) wie in Anspruch 4 beansprucht, **dadurch gekennzeichnet, dass** der zweite Verbindungsabschnitt dadurch realisiert ist, dass der erste Implantatkörper (3) mindestens eine Einkerbung (31, 33) in dem ersten proximalen Ende (3A) und/oder mindestens eine Einkerbung in dem zweiten proximalen Ende (5A) einschließt.

9. Ein chirurgisches Instrument (34) zur Verwendung beim Platzieren eines Zwischenwirbelimplantats gemäß einem der Ansprüche 1 bis 8 zwischen zwei Wirbel (57, 59) und/oder Korrigieren eines Gleitens von Wirbeln (57, 59), wobei das Instrument (34) ein proximales Ende (35), ein distales Ende (37), das von dem proximalen Ende (35) in einer Längsrichtung beabstandet ist, einen Griff (36), der am proximalen Ende (35) liegt, einen Manipulatorabschnitt (38), der am distalen Ende (37) liegt, und eine hohle Stange (39), die sich entlang der Längsrichtung von dem Griff (36) zu dem Manipulatorabschnitt (38) erstreckt, umfasst, und wobei
- der Manipulatorabschnitt (38) ein drehbares Werkzeug mit einem ersten Kopplungsabschnitt (41) umfasst, der ein Koppeln an einen passenden ersten Verbindungsabschnitt am proximalen Ende (11) eines Gewindeelements (7) eines Zwischenwirbelimplantats (1, 101) und eine Übertragung einer Drehbewegung um eine Drehachse, die sich in Längsrichtung des Gerätes (34) erstreckt, auf das Gewindeelement (7) des Zwischenwirbelimplantats (1, 101) erlaubt, und ein Sicherungselement (43), das für den Eingriff eines passenden Sicherungsmittels am proximalen Ende (11) des Gewindeelements (7) des Zwischenwirbelimplantats (1, 101) ausgestaltet ist, um das Werkzeug vor einem Wegbewegen vom proximalen Ende (11) des Gewindeelements (7) entlang einer Längsrichtung zu schützen.

10. Das chirurgische Instrument (34) wie in Anspruch 9 beansprucht, in dem der erste Kopplungsabschnitt (41) die Form eines männlichen oder weiblichen Profils zum Antreiben einer Schraube aufweist.

11. Das Operationsgerät (34) wie in Anspruch 9 oder Anspruch 10 beansprucht, in dem das Sicherungselement ein Gewindestift (43) ist, der an dem ersten Kopplungsabschnitt (41) liegt und in Längsrichtung des Instruments (34) ausgerichtet ist, oder eine Hülse mit einem Gewindesackloch, das in Längsrichtung des Instruments (34) ausgerichtet ist, wobei die Hülse an dem ersten Kopplungsabschnitt (41) liegt, oder das Sicherungselement ist ein männlicher oder weiblicher Teil eines Bajonettverschlusses.

12. Ein chirurgisches Instrument (34) wie in einem der Ansprüche 9 bis 11 beansprucht, zur Verwendung beim Platzieren eines Zwischenwirbelimplantats (1, 101) gemäß einem der Ansprüche 1 bis 8 zwischen zwei Wirbel (57, 59) und/oder Korrigieren eines Gleitens von Wirbeln (57, 59), weiterhin umfassend einen zweiten Kopplungsabschnitt (53A, 53B, 153A, 153B), der ein Koppeln an einen passenden zweiten Verbindungsabschnitt des Implantats (1, 101) erlaubt.

13. Das chirurgische Instrument (34) wie in Anspruch 12 beansprucht, in dem der zweite Kopplungsabschnitt durch mindestens einen Vorsprung (53A, 53B, 153A, 153B) des Manipulatorabschnitts (38) gebildet wird, der in Längsrichtung vorsteht und von dem Werkzeug (41) in einer Richtung rechtwinklig zur Längsrichtung beabstandet ist.

14. Das chirurgische Instrument (34) wie in Anspruch 13 beansprucht, in dem der Kopplungsabschnitt durch einen ersten Vorsprung (53A, 153A) und einen zweiten Vorsprung (53B, 153B) des Manipulatorabschnitts (38) gebildet ist, die beide in Längsrichtung vorstehen und von dem Werkzeug (41) in einer Richtung rechtwinklig zur Längsrichtung beabstandet sind, und die an gegenüberliegenden Seiten des Werkzeugs (41) liegen und bei denen die Breite des ersten Vorsprungs (53A, 153A) sich von der Breite des zweiten Vorsprungs (53B, 153B) unterscheidet.

## Revendications

1. Implant intervertébral (1, 101) s'étendant le long d'une direction longitudinale et comprenant :
- un premier corps d'implant (3, 103) qui s'étend le long d'une direction longitudinale de l'implant (1, 101) et comprend une première extrémité proximale (3A, 103A) et une première extrémité distale (3B, 103B) formant des extrémités opposées du premier corps d'implant (3, 103) dans la direction longitudinale, ledit implant intervertébral comprenant un second corps d'implant (5, 105) qui s'étend le long de la direction longitudinale de l'implant (1, 101) et comprend une seconde extrémité proximale (5A, 105A) et une seconde extrémité distale (5B, 105B) formant des extrémités opposées du second corps d'implant (5, 105) dans la direction longitudinale, où le premier corps d'implant (3, 103) et le second corps d'implant (5, 105) sont reliés de manière telle, qu'ils puissent être mobiles l'un par rapport à l'autre le long de la direction longitudinale ;
- un élément fileté (7) qui est logé dans le premier corps d'implant (3, 103) de manière à pouvoir tourner autour d'un axe de rotation s'étendant dans la direction longitudinale de l'implant (1, 101) et qui a une extrémité proximale (11) qui est accessible de l'extérieur de l'implant (1, 101), où l'extrémité proximale (11) de l'élément fileté (7) comprend une première interface (17, 117) ayant une conception qui permet la transmission d'un mouvement de rotation autour de l'axe de rotation, à l'élément fileté (7), grâce à l'utilisation d'un outil rotatif ; et
- un filetage (15) placé dans le second corps d'implant (5, 105), lequel filetage (15) s'étend dans la direction longitudinale de l'implant (1, 101) et est agencé de manière à coopérer avec l'élément fileté (7) logé dans le premier corps d'implant (3, 103), de manière telle que l'élément fileté (7) fournisse un mouvement longitudinal au second corps d'implant (5, 105) par rapport au premier corps d'implant (3, 103), au cours de la rotation de l'élément fileté (7) autour de son axe de rotation ;
**caractérisé**
**en ce que** l'extrémité proximale (11) de l'élément fileté (7) comprend en outre un moyen de blocage (23, 123) qui permet l'engagement d'un élément de blocage d'accouplement d'un outil utilisé pour transmettre un mouvement de rotation à l'élément fileté (7), de manière à bloquer l'extrémité proximale (11), en l'empêchant de glisser hors de l'outil dans la direction longitudinale.

2. Implant intervertébral (1, 101) selon la revendication 1, **caractérisé en ce que** la première interface (17, 117) se présente sous la forme d'un profil mâle ou femelle d'entraînement de la vis.

3. Implant intervertébral (1, 101) selon la revendication 1 ou la revendication 2, **caractérisé en ce que** le moyen de blocage est un trou borgne fileté (21) placé dans la première interface (17) qui est alignée sur la direction longitudinale de l'implant (1, 101), ou bien est une vis filetée sans tête qui est placée au niveau de la première interface (17) et alignée sur la direction longitudinale de l'implant (1, 101), ou bien le moyen de blocage est une partie mâle ou femelle d'un couplage à baïonnette.

4. Implant intervertébral (1, 101) selon l'une quelconque des revendications 1 à 3, comprenant en outre :
- une deuxième interface (25A, 25B, 27A, 27B) ayant une conception qui permet la transmission d'un mouvement de rotation à la totalité de l'implant (1, 101), grâce à l'utilisation d'une interface d'accouplement d'un outil.

5. Implant intervertébral (1) selon la revendication 4, **caractérisé en ce que** la deuxième interface est réalisée par le fait que
- le premier corps d'implant (3, 103) comprend au moins une partie en creux (25A, 25B) située dans une face (3D, 3E) qui s'étend à partir de la première extrémité proximale (3A, 103A) jusqu'à la première extrémité distale (3B, 103B), où la partie en creux (25A, 25B) s'étend dans ladite face (3D, 3E) dans une direction longitudinale, à partir de ladite première extrémité proximale (3A, 103A) vers ladite première extrémité distale (3B, 103B) ; et/ou
- le second corps d'implant (5, 105) comprend au moins une partie en creux (27A, 27B) située dans une face (5D, 5E) qui s'étend à partir de la seconde extrémité proximale (5A, 105A) jusqu'à la seconde extrémité distale (5B, 105B), où la partie en creux (27A, 27B) s'étend dans ladite face (5D, 5E) dans la direction longitudinale, à partir de ladite seconde extrémité proximale (5A, 105A) vers ladite seconde extrémité distale (5B, 105B).

6. Implant intervertébral (1) selon la revendication 5, **caractérisé en ce que**
- le premier corps d'implant (3) comprend une première face latérale (3D) qui s'étend à partir de la première extrémité proximale (3A) jusqu'à la première extrémité distale (3B), et une seconde face latérale (3E) qui s'étend à partir de la première extrémité proximale (3A) jusqu'à la première extrémité distale (3B) et est placée à l'opposé de la première face latérale (3D), où une partie en creux (25A, 25B) est présente dans chaque face latérale, à savoir la première face (3D) et la seconde face (3E) du premier corps d'implant (3) ;
- le second corps d'implant (5) comprend une première face latérale (5D) qui s'étend à partir de la seconde extrémité proximale (5A) jusqu'à la seconde extrémité distale (5B), et une seconde face latérale (5E) qui s'étend à partir de la seconde extrémité proximale (5A) jusqu'à la seconde extrémité distale (5B) et est placée à l'opposé de la première face latérale (5D), où une partie en creux (27A, 27B) est présente dans chaque face latérale, à savoir la première face (5D) et la seconde face (5E) du second corps d'implant (5) ; et
- chacune des parties en creux (25A, 25B, 27A, 27B) situées dans les faces latérales respectives (3D, 3E, 5D, 5E) commence au niveau de la première et de la seconde extrémité proximale respective (3A, 5A) et s'étend dans une direction longitudinale vers la première et la seconde extrémité distale respective (3B, 5B).

7. Implant intervertébral (1) selon la revendication 6, **caractérisé**
- **en ce que** la partie en creux (25A) située dans la première face latérale (3D) du premier corps d'implant (3) et la partie en creux (27A) située dans la première face latérale (5D) du second corps d'implant (5) ont chacune la même profondeur et un côté ouvert, où les côtés ouverts sont ouverts vers l'autre partie en creux respective,
- **en ce que** la partie en creux (25B) située dans la seconde face latérale (3E) du premier corps d'implant (3) et la partie en creux (27B) située dans la seconde face latérale (3E) du second corps d'implant (5) ont chacune la même profondeur et un côté ouvert, où les côtés ouverts sont ouverts vers l'autre partie en creux respective,
- **en ce que** la largeur combinée de la partie en creux (25A) située dans la première face latérale (3D) du premier corps d'implant (3), et de la partie en creux (27A) située dans la première face latérale (5D) du second corps d'implant (5), diffère de la largeur combinée de la partie en creux (25B) située dans la seconde face latérale (3E) du premier corps d'implant (3), et de la partie en creux (27B) située dans la seconde face latérale (5E) du second corps d'implant (5).

8. Implant intervertébral (1) selon la revendication 4, **caractérisé en ce que** la deuxième interface est réalisée par le fait que le premier corps d'implant (3) comprend au moins une encoche (31, 33) située dans la première extrémité proximale (3A) et/ou au moins une encoche située dans la seconde extrémité proximale (5A).

9. Instrument chirurgical (34) utilisé pour placer un implant intervertébral, selon l'une quelconque des revendications 1 à 8, entre deux vertèbres (57, 59) et/ou pour corriger un glissement de vertèbres (57, 59), où l'instrument (34) comprend une extrémité proximale (35), une extrémité distale (37) qui est espacée de l'extrémité proximale (35) dans une direction longitudinale, un manche (36) placé au niveau de l'extrémité proximale (35), une section de manipulateur (38) placée au niveau de l'extrémité distale (37), et comprend une tige creuse (39) s'étendant le long de la direction longitudinale, à partir du manche (36) jusqu'à la section de manipulateur (38), et où
- la section de manipulateur (38) comprend un outil rotatif ayant une première interface de couplage (41) qui permet le couplage à une première interface d'accouplement au niveau de l'extrémité proximale (11) d'un élément fileté (7) d'un implant intervertébral (1, 101), et qui permet le transfert, à l'élément fileté (7) de l'implant intervertébral (1, 101), d'un mouvement de rotation autour d'un axe de rotation s'étendant dans une direction longitudinale de l'instrument (34), et comprend un élément de blocage (43) qui est conçu pour engager un moyen de blocage d'accouplement au niveau de l'extrémité proximale (11) de l'élément fileté (7) de l'implant intervertébral (1, 101), de façon à bloquer l'outil, en l'empêchant de s'éloigner de l'extrémité proximale (11) de l'élément fileté (7), le long d'une direction longitudinale.

10. Instrument chirurgical (34) selon la revendication 9, dans lequel la première interface de couplage (41) se présente sous la forme d'un profil mâle ou femelle d'entraînement de la vis.

11. Instrument chirurgical (34) selon la revendication 9 ou la revendication 10, dans lequel l'élément de blocage est une vis filetée sans tête (43) qui est placée au niveau de la première interface de couplage (41) et alignée sur la direction longitudinale de l'instrument (34), ou bien est une douille ayant un trou borgne fileté qui est aligné sur la direction longitudinale de l'instrument (34), où la douille est placée au niveau de la première interface de couplage (41), ou bien l'élément de blocage est une partie mâle ou femelle d'un couplage à baïonnette.

12. Instrument chirurgical (34) selon l'une quelconque des revendications 9 à 11, utilisé pour placer un implant intervertébral (1, 101), selon l'une quelconque des revendications 1 à 8, entre deux vertèbres (57, 59) et/ou pour corriger un glissement de vertèbres (57, 59), ledit instrument chirurgical comprenant en outre :
- une deuxième interface de couplage (53A, 53B, 153A, 153B) qui permet un couplage à une deuxième interface d'accouplement de l'implant (1, 101).

13. Instrument chirurgical (34) selon la revendication 12, dans lequel la deuxième interface de couplage est formée par au moins une partie saillante (53A, 53B, 153A, 153B) de la section de manipulateur (38), partie saillante qui dépasse dans la direction longitudinale et est espacée de l'outil (41) dans une direction perpendiculaire à la direction longitudinale.

14. Instrument chirurgical (34) selon la revendication 13, dans lequel l'interface de couplage est formée par une première partie saillante (53A, 153A) et par une deuxième partie saillante (53B, 153B) de la section de manipulateur (38), parties saillantes qui dépassent toutes les deux dans la direction longitudinale et qui sont espacées de l'outil (41) dans une direction perpendiculaire à la direction longitudinale, et qui sont placées au niveau de côtés opposés de l'outil (41), outil dans lequel la largeur de la première partie saillante (53A, 153A) diffère de la largeur de la deuxième partie saillante (53B, 153B).
